# EUROPÄISCHE PATENTSCHRIFT

(11) **EP 3 180 104 B1**
(45) Veröffentlichungstag und Bekanntmachung des Hinweises auf die Patenterteilung: **06.06.2018**
(21) Anmeldenummer: 16763791.7
(22) Anmeldetag: 09.09.2016
(51) Int. Cl.: B01D 53/22, B01D 53/30, C10L 3/10

(54) **VORRICHTUNG UND VERFAHREN ZUR AUFTRENNUNG EINES GASGEMISCHES IN EINER MEMBRANEINHEIT**
DEVICE AND METHOD OF SEPARATING A GAS MIXTURE IN A MEMBRANE UNIT
DISPOSITIF ET PROCÉDÉ DESTINÉS A LA SEPARATION D'UN MELANGE GAZEUX PAR UNE UNITÉ DE MEMBRANE

(30) Priorität: 10.09.2015 EP 15184639
(43) Veröffentlichungstag der Anmeldung: 21.06.2017
(73) Patentinhaber: Axiom Angewandte Prozeßtechnik Ges. m.b.H., 2443 Ebreichsdorf (AT)
(72) Erfinder: SZIVACZ, Johannes, 2443 Ebreichsdorf (AT); WINTERSPERGER, Johannes, 2443 Ebreichsdorf (AT)
(74) Vertreter: Sonn & Partner Patentanwälte
(86) Internationale Anmeldenummer: PCT/EP2016/071261
(87) Internationale Veröffentlichungsnummer: WO 2017/042310

(56) Entgegenhaltungen:
- EP-A1- 2 679 297
- EP-A1- 2 762 220
- WO-A1-2010/141963

## Beschreibung

Die vorliegende Erfindung betrifft eine Vorrichtung und ein Verfahren zur Auftrennung eines Gasgemisches in Produktgas und Offgas mittels Gaspermeation.

Bei Gaspermeationsmembraneinheiten erfolgt die Trennung von Produktgas und Offgas mittels Permeation, wobei ein beispielsweise an Produktgas angereichertes Retentat und ein an Offgas angereichertes Permeat gewonnen werden können. Die Konzentrationen an Produktgas im Retentat und Offgas im Permeat sind unter anderem von den jeweils angewendeten Prozess-Parametern abhängig, generell ist für eine erhöhte Produktgas-Qualität immer ein erhöhter Energieeinsatz erforderlich (wegen höherem Druck, niedrigerer Ausbeute in Bezug auf eingesetztes Feedgas, etc.). Verbesserte Verfahren zur Steigerung der Produktgas-Ausbeute oder zur effizienteren Nutzung von Energie im Zuge eines derartigen Verfahrens sind daher wünschenswert. Auch ist es wünschenswert, die bei der Errichtung einer Gaspermeationsanlage anfallenden Investitionskosten möglichst gering zu halten.

Bisher sind Vorrichtungen zur Auftrennung eines Gasgemisches in Produktgas und Offgas mittels Gaspermeation derart aufgebaut, dass das druckbeaufschlagte Feedgas in einer Membraneinheit in das Retentat und in das Permeat aufgetrennt werden, wobei beispielsweise das Retentat das Produktgas und das Permeat das Offgas enthält. Nachteil dieser einstufigen Lösung sind niedrige Produktgasqualität und niedrige Produktgasausbeute, die mit einem erhöhten Energiebedarf verbunden ist. Weiters ist diese Vorrichtung nur für sehr selektive Membranen wirtschaftlich einsetzbar.

Verbesserte Vorrichtungen zur Auftrennung eines Gasgemisches in Produktgas und Offgas mittels Gaspermeation sind derart aufgebaut, dass das Permeat einer ersten Membraneinheit druckbeaufschlagt als Feedgas für eine zweite Membraneinheit verwendet wird, wobei die Retentatströme der beiden Membraneinheiten das Produktgas und der Permeatstrom der zweiten Membraneinheit das Offgas enthalten. Der Anlage kann gegebenenfalls noch ein Verdichter vorgeschaltet werden, wenn das Feedgas nicht druckbeaufschlagt vorhanden ist. Der Vorteil dieser Vorrichtung ist eine verbesserte Produktgasausbeute. Nachteil dieser Lösung sind die weiterhin niedrige Produktgasqualität und ein aufgrund der erforderlichen Verdichtung des Gases für die zweite Membraneinheit erhöhter Energiebedarf. Weiters ist diese Vorrichtung nur für sehr selektive Membranen wirtschaftlich einsetzbar.

Weiters sind Vorrichtungen bekannt, wobei das Retentat einer ersten Membraneinheit als Feedgas einer zweiten Membraneinheit verwendet wird, das Permeat der zweiten Membraneinheit dem druckbeaufschlagten Feedgas der ersten Membraneinheit beigemischt wird, das Retentat der zweiten Membraneinheit als Produktgas und das Permeat der ersten Membraneinheit als Offgas abgezogen wird. Da hier das Permeat der zweiten Membraneinheit sozusagen im Kreislauf geführt wird, muss die Dimension der Anlage und aller nötigen Teile (Kompressoren, Leitungen, Membraneinheiten, Kälteabscheider, Schwefelfeinabscheider etc.) entsprechend dem Volumsstrom des im Kreislauf geführten Permeats der ersten Membraneinheit vergrößert werden. Bei einem angenommenen Volumsstrom an Feedgas von 100 m³/h und Beimischung von 80 m³/h an Permeat der zweiten Membraneinheit zu diesem Feedgas ergibt sich vor dem Kompressor ein Gesamtvolumsstrom von 180 m³/h, gemäß welchem die Anlage zu dimensionieren ist. Vorteil dieses Verfahren ist, dass eine höhere Ausbeute an Produktgas erzielt werden kann, auch können aufgrund der zweistufigen Ausführung weniger selektive Membranen eingesetzt werden, nachteilig ist dabei die um den Faktor 1,2 bis 2,5 nötige übergroße Auslegung der Anlage und ein aufgrund der Rückführung erhöhter Energiebedarf.

Die US 4,130,403 A (D1) offenbart die Rückkopplung des Retentatausgangs einer Membraneinheit an die Gaszufuhr einer weiteren Membraneinheit, wobei dieser lediglich eine einzige Membraneinheit vorgeschaltet ist.

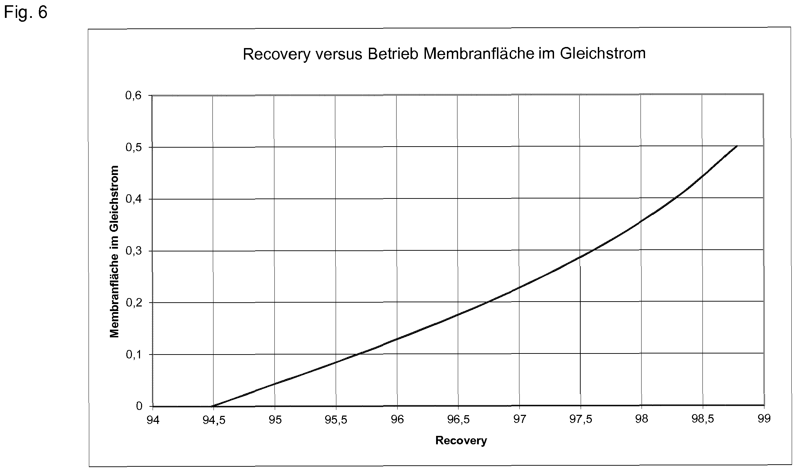

Agrawal R et al. (Journal of Membran Science, Elsevier Scientific Publ. Company, Bd. 112, Nr. 2) ist auf kaskadierte Anordnungen mit zwei Kompressoren gerichtet. Fig. 6 zeigt Rückkopplungen an den Gaseingang der jeweils ersten Membraneinheit.

Die FR 2 917 305 A1 (D3) offenbart eine Matrixanordnung einer Vielzahl von Membraneinheiten.

Aus der WO 2010/141963 A1 ist eine Vorrichtung zur Auftrennung eines Gasgemisches in Produktgas und Offgas mittels Gaspermeation mit mindestens zwei Membraneinheiten (1) und (2) und einem der ersten Membraneinheit (1) vorgeschalteten Verdichter (3) bekannt, welche Membraneinheiten (1) und (2) einen Gaseingang (1a, 2a), einen Retentatausgang (1b, 2b) und einen Permeatausgang (1c, 2c) aufweisen, wobei der Retentatausgang (1b) der ersten Membraneinheit (1) mit dem Gaseingang (2a) der zweiten Membraneinheit (2), der Permeatausgang (2c) der zweiten Membraneinheit (2) ansaugseitig mit dem Verdichter (3) bzw. der in den Verdichter führenden Gaszufuhr und der Verdichter (3) mit dem Gaseingang (1a) der ersten Membraneinheit (1) leitungsmäßig verbunden ist, Produktgas über Retentatausgang (2b) und Offgas über Permeatausgang (1c) erhalten wird.

Bei einer solchen Vorrichtung ist gemäß der WO 2010/141963 A1 vorgesehen, dass der Permeatausgang (4c) einer vorgeschalteten Membraneinheit (4) mit der Gaszufuhr des Verdichters (3) leitungsmäßig verbunden ist, wobei der Membraneinheit (4) noch mindestens eine weitere Membraneinheit (5) durch leitungsmäßige Verbindung des Retentatausgangs (5b) der weiteren Membraneinheit (5) mit dem Gaseingang (4a) der Membraneinheit (4) vorgeschaltet ist und zusätzliches Produktgas über Retentatausgang (4b) und zusätzliches Offgas über Permeatausgang (5c) erhalten wird.

All diesen Vorrichtungen gemeinsam ist, dass die einzelnen Membraneinheiten immer im Gegenstrom betrieben werden.

Aus der WO 2010/141963 A1 sind Membraneinheiten bekannt, die einen Gaseingang, einen Retentatausgang und zwei Permeatausgänge aufweisen, wobei die Permeaträume solcher Membraneinheiten jeweils im Bereich zwischen den beiden Permeatausgängen durch eine Wand abgetrennt sein können. Solche Membraneinheiten sollen eine erhöhte Produktgas-Ausbeute ermöglichen.

Die Aufgabe der vorliegenden Erfindung besteht nun darin, eine Vorrichtung und ein Verfahren zur Auftrennung eines Gasgemisches in Produktgas und Offgas mittels Gaspermeation zur Verfügung zu stellen, welche eine möglichst geringe, durch Steuerung der Vorrichtung einstellbare Konzentration von Produktgas im Offgas sowie einen effizienteren Energieeinsatz ermöglicht.

Die Erfindung geht dabei von einer Vorrichtung zur Auftrennung eines Gasgemisches in Produktgas und Offgas mittels Gaspermeation mit einer Membraneinheit (1) und einem der Membraneinheit (1) vorgeschalteten, vorzugsweise drehzahlregelbaren Verdichter (3) aus, welche Membraneinheit (1) einen Gaseingang (1a), einen Retentat- bzw. Produktgasausgang (1b) und einen Permeat- bzw. Offgasausgang (1c) aufweist, wobei die Membraneinheit (1) mindestens einen weiteren, vom Gaseingang (1a) stromabwärts liegenden Permeatausgang (1c') aufweist und der Permeatausgang (1c') der Membraneinheit (1) ansaugseitig mit dem Verdichter (3) bzw. der in den Verdichter führenden Gaszufuhr leitungsmäßig verbunden ist. Dabei ist erfindungsgemäß vorgesehen, dass am oder nach dem Retentatausgang (1b) eine Druckregeleinrichtung (2) vorgesehen ist und ein Gasgemisch mit einem Anteil von mehr als 50 Vol.-%, vorzugsweise mehr als 55 Vol.-%, 60 Vol.-%, 65 Vol.-%, 70 Vol.-%, 75 Vol.-%, 80 Vol.-%, 85 Vol.-%, insbesondere bevorzugt mehr als 90 Vol.-% bzw. 95 Vol.-% CH_{4/}CO₂ im Gasgemisch und mit einer Methankonzentration nicht über 30 Vol.-% eingesetzt wird, wobei der Abzug des Permeats über den Permeatausgang (1c') in Abhängigkeit von der Konzentration von Produktgas im Offgas gesteuert bzw. geregelt wird. Durch diese Kombination von Druckregeleinrichtung und bestimmtem Gasgemisch (wobei "hauptsächlich" für den Zweck der vorliegenden Erfindung einen Anteil im Gasgemisch von mehr als 50 Vol.-%, vorzugsweise mehr als 55 Vol.-%, 60 Vol.-%, 65 Vol.-%, 70 Vol.-%, 75 Vol.-%, 80 Vol.-%, 85 Vol.-%, insbesondere bevorzugt mehr als 90 Vol.-% bzw. 95 Vol.-%, bedeutet), welche einerseits die Methankonzentration im Feedgas beschränkt und gleichzeitig durch die Druckregeleinrichtung für das Retentat einen Widerstand im Retentatraum vorsieht und beispielsweise durch ein Ventil oder Stellventil, die Ansaugseite eines vorzugsweise drehzahlregelbaren Verdichters oder (ebenfalls vorzugsweise drehzahlregelbaren) Kompressors oder eine Zuleitung in eine Stufe eines vorzugsweise drehzahlregelbaren mehrstufigen Verdichters oder Kompressors dargestellt sein kann, ist es möglich, die Druckregeleinrichtung den Vorgaben entsprechend mehr oder weniger zu schliessen bzw. einen Widerstand durch die Drehzahl des Verdichters oder Kompressors zu geben oder durch Regelung bzw. Steuerung der Drehzahl des Verdichters oder Kompressors einzustellen. Ein Schließen des Regelventils (2) ist dabei nicht notwendigerweise mit einer Druckerhöhung im Retentatraum verbunden, so kann beispielsweise zusammen mit der Schließung des Regelventils (2) eine Drehzahlminderung im Kompressor (3) einhergehen, wodurch der Druck im Retentatraum letztendlich gleich bleibt und nicht erhöht wird. Wichtig ist nur, dass in der erfindungsgemäßen Membraneinheit retentatseitige ein Widerstand für das dort vorhandene Gasgemisch vorgesehen wird. Dadurch kann die Membraneinheit im Bereich des zweiten, vom Gaseingang stromabwärts gelegenen Permeatausgang (1c') zumindest teilweise kontrolliert im Gleichstrom betrieben werden, während die Membraneinheit im Bereich des ersten Permeatausgangs, wie üblich, vorwiegend im Gegenstrom betrieben wird. "Zumindest teilweise" bzw." vorwiegend" bedeuten dabei nicht, dass an beiden Permeatausgängen zugleich Gleichstrom und Gegenstrom herrscht (was technisch unmöglich ist), sondern dass das am jeweiligen Permeatausgang austretende Permeat aus einem Bereich der Membran stammt, welcher, je nach den gewählten Bedingungen, eben permeatseitig entweder in Gleich- oder im Gegenstrom zur retentatseitigen Strömungsrichtung betrieben wurde. Daraus ergeben sich überraschenderweise wesentliche Verbesserungen beim Betrieb der erfindungsgemäßen Vorrichtung, hauptsächlich hinsichtlich einer Verringerung der Konzentration an Produktgas im Offgas aber auch hinsichtlich einer Erhöhung der Menge an Produktgas. So kann erfindungsgemäß bei der Trennung eines hauptsächlich aus CH_{4/}CO₂ bestehenden Gasgemisches, beispielsweise Schwachgas, Grubengas oder Biogas, in CH₄ als Produktgas und hauptsächlich CO₂ als Offgas mittels der erfindungsgemäßen Vorrichtung eine überraschend hohe Recovery an CH₄ im Produktgas von mehr als 98%, vorzugsweise bis zu 99,8 % erreicht werden, wenn die Methankonzentration im Feedgas wie erfindungsgemäß vorgesehen nicht über 30 Vol.-% liegt. Selbstverständlich kann, beispielsweise bei Vorsehen von Sensoren an geeigneter Stelle in der erfindungsgemäßen Vorrichtung, die Druckregeleinrichtung auch automatisiert betrieben werden, indem die Druckregeleinrichtung mittels einer Steuereinrichtung zur Einhaltung eines vorgegebenen, an einem oder mehreren Stellen innerhalb oder außerhalb der erfindungsgemäßen Vorrichtung gemessenen Grenzwertes geöffnet bzw. geschlossen wird. Der Grenzwert kann beispielsweise die Methankonzentration im Offgas sein, wobei in dem Fall der Sensor ein an oder nahe beim Permeatausgang (1c) vorgesehener Gassensor sein kann. Beispielsweise ist für die Freisetzung von Methan (Produktgas bzw. Retentat bei der Trennung von Biogas mittels Gaspermeation) in Deutschland die maximal emittierbare Methanmenge im Abgas auf 0,2 Vol.-% der im Biogasprozess produzierten Methanmasse begrenzt, wobei das Einhalten dieses Grenzwerts beispielsweise durch die erfindungsgemäße Vorrichtung bei Vorsehen einer Steuereinrichtung auch automatisiert vorgesehen werden kann. Dadurch, dass der Permeatausgang (1c') der Membraneinheit (1) ansaugseitig mit dem Verdichter (3) bzw. der in den Verdichter führenden Gaszufuhr leitungsmäßig verbunden ist wird vorgesehen, dass das Permeat dem Feedgas vor oder während seiner Druckbeaufschlagung zugeführt werden kann. Sofern die Zufuhr ein höheres Druckniveau erfordert, kann dieses durch gegebenenfalls zusätzlich vorgesehene Verdichter oder Kompressoren (auf welche im Folgenden nicht ausdrücklich Bezug genommen wird) bewerkstelligt werden. Idealerweise kann ein die erfindungsgemäße Vorrichtung über den Permeatausgang (1c) verlassendes Offgas (CO₂) ohne weitere Reinigung bezüglich des enthaltenen Methans direkt emittiert bzw. weiterverwendet werden.

Gemäß einer bevorzugten Ausführungsform der vorliegenden Erfindung ist vorgesehen, dass am oder nach dem ersten Permeatausgang (1c) ein vorzugsweise drehzahlregelbarer Verdichter (4) und/oder eine Druckregeleinrichtung (5) vorgesehen ist bzw. sind. Durch das Vorsehen einer Druckregeleinrichtung, beispielsweise eines Ventils oder Stellventils, kann bei Verringerung des Durchflusses durch das Ventil im Permeatraum der erfindungsgemäßen Vorrichtung ein Rückstau erzeugt werden, wodurch sich die im Gleichstrom betriebene Fläche der Trennmembran vergrößert. Wird andererseits am oder nach dem ersten Permeatausgang (1c) ein vorzugsweise drehzahlregelbarer Verdichter betrieben, erhöht sich der Abzug von Offgas aus dem ersten Permeatausgang (1c) und die im Gleichstrom betriebene Fläche der Trennmembran verringert sich. Beim Vorsehen von sowohl Druckregeleinrichtung als auch vorzugsweise drehzahlregelbaren Verdichter kann die erfindungsgemäße Vorrichtung somit in jede Richtung gesteuert werden.

Günstig ist auch, wenn in der Leitung vom Permeatausgang (1c') der Membraneinheit (1) ansaugseitig zum Verdichter (3) bzw. der in den Verdichter führenden Gaszufuhr eine Druckregeleinrichtung (6) vorgesehen ist. Ähnlich wie bei der zuvor beschriebenen bevorzugten Ausführungsform kann durch das Vorsehen eine Druckregeleinrichtung in der Leitung vom Permeatausgang (1c') der Membraneinheit (1) ansaugseitig zum Verdichter (3) bzw. der in den Verdichter führenden Gaszufuhr der Rückstau in den Permeatraum der erfindungsgemäßen Vorrichtung vergrößert werden (wenn die Druckregeleinrichtung, beispielsweise ein Ventil oder Stellventil, den Gasdurchfluss verringert), wodurch sich die im Gleichstrom betriebene Fläche der Trennmembran verringert. Wenn andererseits die Druckregeleinrichtung geöffnet wird, ergibt sich aus der Ansaugleistung des Verdichters (3) im Permeatraum zumindest im Bereich des Permeatausgangs (1c') ein Druckverlust, wodurch sich die im Gleichstrom betriebene Fläche der Trennmembran vergrößert. Unter den Schutzbereich der vorliegenden Erfindung fällt jedenfalls auch eine Ausführungsform, bei welcher die Leitung vom Permeatausgang (1c') der Membraneinheit (1) ansaugseitig in einen Verdichter bzw. der in den Verdichter führenden Gaszufuhr einer parallelen Membraneinheit führt. Dabei ist in der Leitung vorzugsweise ebenfalls die Druckregeleinrichtung (6) vorgesehen, welche in Abhängigkeit von der Konzentration von Produktgas im Offgas gesteuert bzw. geregelt wird.

Erfindungsgemäß wird auch vorgesehen, dass der drehzahlregelbare Verdichter (3) und die Druckregeleinrichtung (2) mit einer Steuereinrichtung (7) verbunden sind. Bei Vorsehen von beispielsweise einem Gassensor im Bereich des Permeatausgangs (1c), welcher Gassensor beispielsweise die Konzentration an Produktgas im Offgas direkt oder indirekt messen kann, ist es wie bereits zuvor erwähnt möglich, die erfindungsgemäße Vorrichtung derart zu betreiben, dass ein vorgegebener Produktgasanteil im Offgas nicht überschritten wird.

Eine weitere bevorzugte Ausführungsform der vorliegenden Erfindung ist dadurch gekennzeichnet, dass der vorzugsweise drehzahlregelbare Verdichter (4) und/oder die Druckregeleinrichtung (5) mit einer bzw. der Steuereinrichtung (7) verbunden sind. Bei Vorsehen von beispielsweise einem Gassensor im Bereich des Permeatausgangs (1c), welcher Gassensor beispielsweise die Konzentration an Produktgas im Offgas direkt oder indirekt messen kann, ist es wie bereits zuvor erwähnt möglich, die erfindungsgemäße Vorrichtung derart zu betreiben, dass ein vorgegebener Produktgasanteil im Offgas nicht überschritten wird.

Dabei ist ebenfalls günstig, wenn die Druckregeleinrichtung (6) mit einer bzw. der Steuereinrichtung (7) verbunden ist. Bei Vorsehen von beispielsweise einem Gassensor im Bereich des Permeatausgangs (1c), welcher Gassensor beispielsweise die Konzentration an Produktgas im Offgas direkt oder indirekt messen kann, ist es wie bereits zuvor erwähnt möglich, die erfindungsgemäße Vorrichtung derart zu betreiben, dass ein vorgegebener Produktgasanteil im Offgas nicht überschritten wird.

Gemäß einem weiteren Aspekt der vorliegenden Erfindung wird die genannte Vorrichtung zur Trennung eines Gasgemisches mit einem Anteil im Gasgemisch von mehr als 50 Vol.-%, vorzugsweise mehr als 55 Vol.-%, 60 Vol.-%, 65 Vol.-%, 70 Vol.-%, 75 Vol.-%, 80 Vol.-%, 85 Vol.-%, insbesondere bevorzugt mehr als 90 Vol.-% bzw. 95 Vol.-% CH_{4/}CO₂ im Gasgemisch mit einer Methankonzentration nicht über 30 Vol.-% in CH₄ als Produktgas und hauptsächlich CO₂ als Offgas eingesetzt. Beispiele für derartige Gasgemisches sind Schwachgas, Grubengas, Biogas et cetera.

Noch ein weiterer Aspekt der vorliegenden Erfindung betrifft ein Verfahren zur Auftrennung eines Gasgemisches mit einem Anteil im Gasgemisch von mehr als 50 Vol.-%, vorzugsweise mehr als 55 Vol.-%, 60 Vol.-%, 65 Vol.-%, 70 Vol.-%, 75 Vol.-%, 80 Vol.-%, 85 Vol.-%, insbesondere bevorzugt mehr als 90 Vol.-% bzw. 95 Vol.-% CH_{4/}CO₂ und mit einer Methankonzentration nicht über 30 Vol.-% in Produktgas und Offgas mittels Gaspermeation, dadurch gekennzeichnet, dass das Permeat einer Membraneinheit (1) teilweise im Gleichstrom und teilweise im Gegenstrom gewonnen wird, wobei das im Gleichstrom gewonnene Permeat druckbeaufschlagt und der Membraneinheit (1) wieder als Feedgas zugeführt wird, das die Gegenstrom gewonnene Permeat als Offgas abgezogen wird, und weiters das Retentat der Membraneinheit (1) über den Retentatausgang (1b) der Membraneinheit (1) und eine dort vorgesehene Druckregeleinrichtung (2) als Produktgas abgezogen wird, wobei der Abzug des im Gleichstrom gewonnenen Permeats in Abhängigkeit von der Konzentration von Produktgas im Offgas gesteuert bzw. geregelt wird. Der Abzug des im Gleichstrom gewonnenen Permeats erfolgt dabei über die genannte Druckbeaufschlagung, wodurch das Permeat der Membraneinheit wieder als Feedgas zugeführt wird.

Vorzugsweise ist dabei vorgesehen, dass der Druck des Retentats in der Membraneinheit (1) erhöht wird. Wie bereits ausgeführt wird beispielsweise durch Vorsehen eines Widerstandes, wie oben hinsichtlich des Widerstandes (2) geoffenbart, der Druck des Retentat in der Membraneinheit erhöht, was in weiterer Folge zu einer Vermehrung des im Gleichstrom gewonnenen Permeats führt.

Die vorliegende Erfindung wird nun unter Bezugnahme auf die beiliegenden Figuren näher erklärt. Dabei zeigen:
Fig. 1 ein Blockschaltbild einer erfindungsgemäßen Vorrichtung mit einer Membraneinheit (1), einer Druckregeleinrichtung (2) und einem der Membraneinheit (1) vorgeschalteten Kompressor bzw. Verdichter (3) ;
Fig. 2 ein Blockschaltbild der Vorrichtung von Fig. 1 mit zusätzlich einer zweiten Druckregeleinrichtung (5) sowie einem zweiten Kompressor bzw. Verdichter (4);
Fig. 3 ein Blockschaltbild der Vorrichtung von Fig. 2 mit zusätzlich einer dritten Druckregeleinrichtung (6) sowie
Fig. 4 ein Blockschaltbild der Vorrichtung von Fig. 3 mit zusätzlich einer Steuereinrichtung (7);
Fig. 5 einen Vergleich zwischen den erzielten Werten bei Betrieb desselben Membraneinheit im Gegenstrom, im Gleichstrom sowie gemäß der vorliegenden Erfindung (mit teilweisem Betrieb im Gleichstrom);
Fig. 6 die hinsichtlich Recovery erzielten Ergebnisse bei stufenloser Veränderung des Anteils an im Gleichstrom betriebene Membranfläche, wobei dieselbe Membraneinheit wie für die Ergebnisse von Figur 5 verwendet wurde und
Fig. 7a und 7b eine Veranschaulichung der erfindungsgemäß verwendeten Begriffe Gleich- und Gegenstrom bzw. "zumindest teilweise" und "vorwiegend".

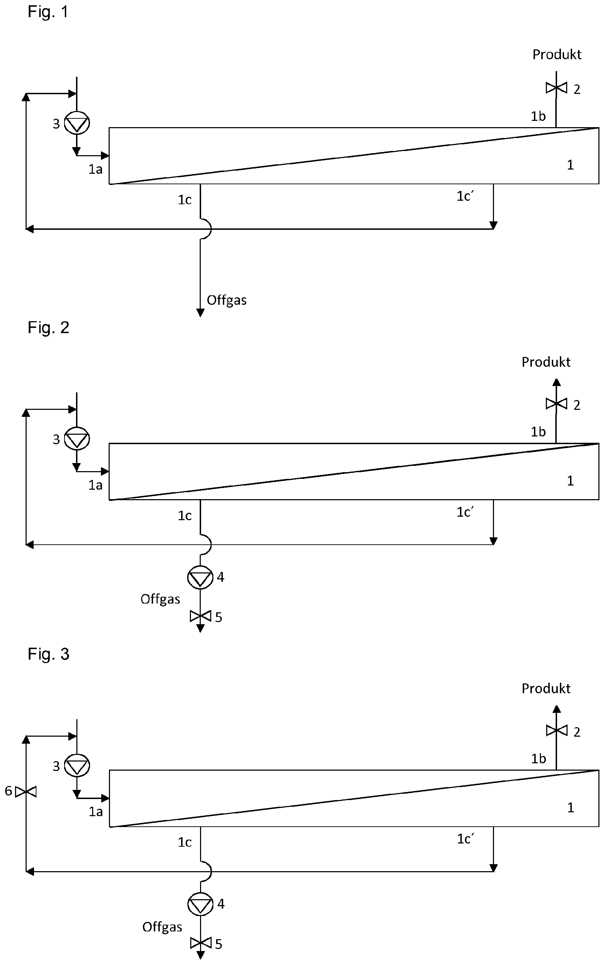

Die Membraneinheit gemäß Fig. 1 weist einen Gaseingang (1a), einen Retentatausgang (1b) und einen zusätzlichen Permeatausgang (1c') auf. In der Membraneinheit (1) wird im Gegenstrombereich anfallendes Offgas über der Permeatausgang (1c) abgeführt, im Gleichstrombereich über Permeatausgang (1c') und mittels des Verdichters (3) abgesaugtes Permeat wird über den Verdichter (3) der Membraneinheit (1) wieder als Feedgas wieder zugeführt. Dadurch wird über Verdichter (3), Gaseingang (1a) und Permeatausgang (1c') in der Membraneinheit (1) im Gleichstrombereich abgetrenntes Offgas im Kreislauf geführt. Produktgas wird über den Retentatausgang (1b) abgeführt, wobei die dort vorgesehene Druckregeleinrichtung (2) dazu dient, den Druck im Retentatraum der Membraneinheit (1) zu erhöhen, wodurch der erfindungsgemäße Betrieb der Trennmembran im teilweisen Gleichstrom erst kontrollierbar wird. Hinsichtlich Fig. 1 gilt unabhängig davon, wo genau die Permeatausgänge zueinander platziert sind (abgesehen davon, dass der Permeatausgang 1c' zwingend stromabwärts vom Gaseingang 1a sein muss) folgendes:
Bezüglich Permeatausgang 1c wird ein vom linken Bereich (vom Ausgang 1c aus gesehen) des Permeatraums stammendes Permeat im Gleichstrom gewonnenen, da die Strömungsrichtung des Retentats von Gaseingang 1a zum Retentatausgang 1b gleich ist mit der Strömungsrichtung vom linken Bereich des Permeatraums zum Permeatausgang 1c. Andererseits wird ein vom rechten Bereich (vom Ausgang 1c aus gesehen) des Permeatraums stammendes Permeat im Gegenstrom gewonnenen, da die Strömungsrichtung des Retentats von Gaseingang 1a zum Retentatausgang 1b entgegengesetzt ist zur Strömungsrichtung vom rechten Bereich des Permeatraums zum Permeatausgang 1c.

Ähnliches gilt bezüglich Permeatausgang 1c', dort wird ein vom linken Bereich (vom Ausgang 1c' aus gesehen) des Permeatraums stammendes Permeat ebenfalls im Gleichstrom gewonnenen, da die Strömungsrichtung des Retentats von Gaseingang 1a zum Retentatausgang 1b gleich ist mit der Strömungsrichtung vom linken Bereich des Permeatraums zum Permeatausgang 1c'. Andererseits wird ein vom rechten Bereich (vom Ausgang 1c' aus gesehen) des Permeatraums stammendes Permeat im Gegenstrom gewonnenen, da die Strömungsrichtung des Retentats von Gaseingang 1a zum Retentatausgang 1b entgegengesetzt ist zur Strömungsrichtung vom rechten Bereich des Permeatraums zum Permeatausgang 1c'. Es versteht sich von selbst, dass an keinem Permeatausgang ein Permeat vorhanden sein kann, welches von Bereichen der Membran stammt, welche ausschließlich in Gleich- bzw. ausschließlich im Gegenstrom betrieben wurden.

In Fig. 2 wird die Ausführungsform von Fig. 1 gezeigt, wobei in der an den Permeatausgang (1c) anschließenden Leitung eine zweite Druckregeleinrichtung (5) sowie ein zweiter, vorzugsweise drehzahlregelbarer Kompressor bzw. Verdichter (4) vorgesehen ist. Bei Schließen der Druckregeleinrichtung (5) staut sich Offgas zurück in den Retentatraum der Membraneinheit (1), wodurch zwangsweise mehr Retentat beim Retentatausgang (1c') austreten muss, wodurch sich die im Gleichstrom betriebene Fläche der Trennmembran vergrößert. Wird andererseits die Druckregeleinrichtung (5) vollständig geöffnet und der vorzugsweise drehzahlregelbare Verdichter (4) gestartet, muss zwangsweise mehr Retentat beim Retentatausgang (1c) austreten, wodurch sich der Abzug von Offgas aus dem ersten Permeatausgang (1c) erhöht und sich die im Gleichstrom betriebene Fläche der Trennmembran verringert.

In Fig. 3 wird die Ausführungsform von Fig. 2 gezeigt, wobei in der Leitung zwischen Permeatausgang (1c') und Verdichter (3) bzw. Zugangsleitung zum Verdichter (3) eine weitere Druckregeleinrichtung (6) vorgesehen ist. Mittels dieser Druckregeleinrichtung (6) kann der Austritt von Permeat aus dem Permeatraum der Membraneinheit (1) über den Permeatausgang (1c') unabhängig von der Leistung des Verdichters (3) verringert werden, wodurch sich die Druckverhältnisse im Permeatraum nahe dem Permeatausgang (1c') verändern. Bei erzeugtem Rückstau drängt das Retentat vermehrt zum Retentatausgang (1c), wodurch sich die im Gleichstrom betriebene Fläche der Trennmembran ebenfalls verringert.

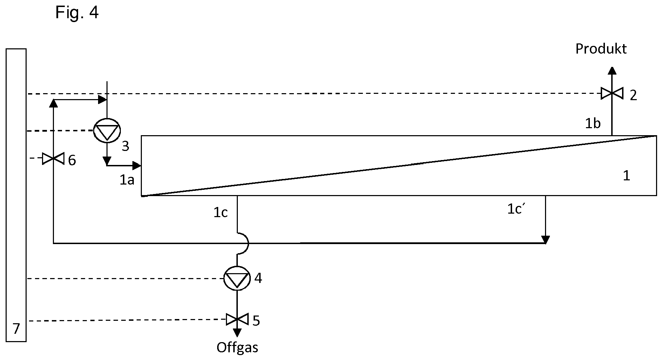

In Figur 4 wird die Ausführungsform von Fig. 3 gezeigt, wobei die Druckregeleinrichtungen (2, 5, 6) und die Verdichter (3, 4) mit einer Steuereinrichtung (7) verbunden sind. Es sei in diesem Zusammenhang ausdrücklich angemerkt, dass erfindungsgemäß auch eine Verbindung von nur einzelnen der Einrichtungen (2, 3, 4, 5, 6) mit der Steuereinrichtung vorgesehen ist. Allfällige für den Betrieb der Steuereinrichtung nötige Sensoren, beispielsweise Gassensoren, Drucksensoren und/oder Durchflusssensoren, werden in den Figuren 1 bis 4 nicht gezeigt. Die Steuereinrichtung (7) ist derart ausgelegt, dass sie das Öffnen und Schließen der Druckregeleinrichtungen (2, 5, 6) unabhängig voneinander sowie eine ebenfalls unabhängige Regelung der Drehzahl der Verdichter (3, 4) vorsehen kann.

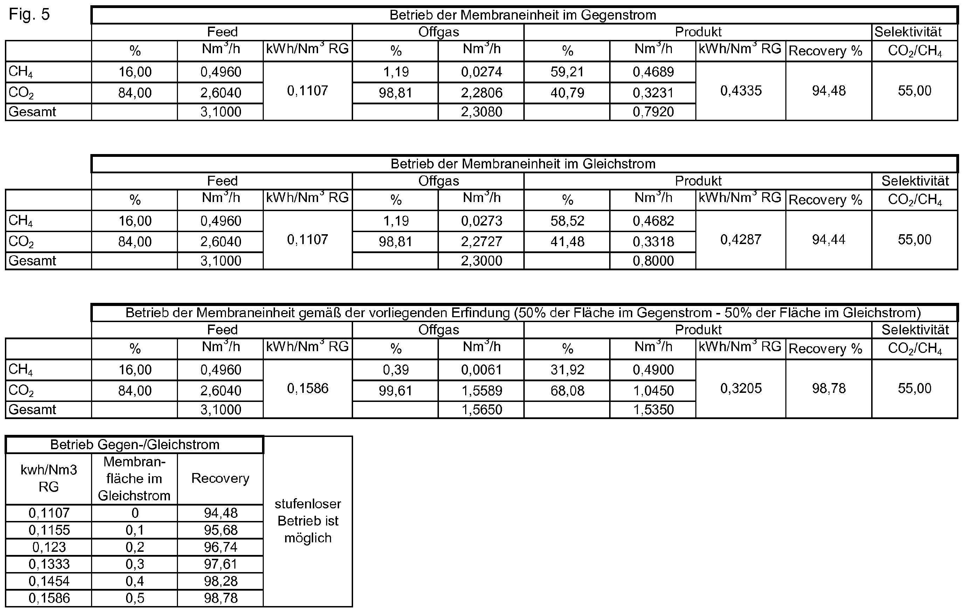

Aus Figur 5 ist ersichtlich, dass sich die Werte für den Betrieb der Membraneinheit im Gegenstrom bzw. im Gleichstrom nur wenig voneinander unterscheiden, erst bei teilweisem Betrieb der Membranfläche im gegen Strom und teilweisem Betrieb im Gleichstrom ist ein deutliches Anwachsen der Recovery erkennbar. Dies ist insofern günstig, als bei Verwendung der erfindungsgemäßen Membraneinheit bzw. Betrieb der Membraneinheit gemäß der vorliegenden Erfindung bei einer Methankonzentration im Feedgas nicht über 30 Vol.% der Methananteil im Offgas auf bisher unerreichte Werte reduziert werden kann, was insbesondere hinsichtlich der Effizienz der Abtrennung von Methan aus dem verwendeten Feedgas wesentliche Vorteile bringt. Es versteht sich, dass die erfindungsgemäße Vorrichtung nicht nur für die Abtrennung von Methan aus einem methanhältigen Feedgas einsetzbar ist sondern, bei Verwendung von für das jeweilige Gasgemisch geeigneten Permeationsmembranen, die Recovery an Produktgas für jegliches Gasgemisch wesentlich steigern kann.

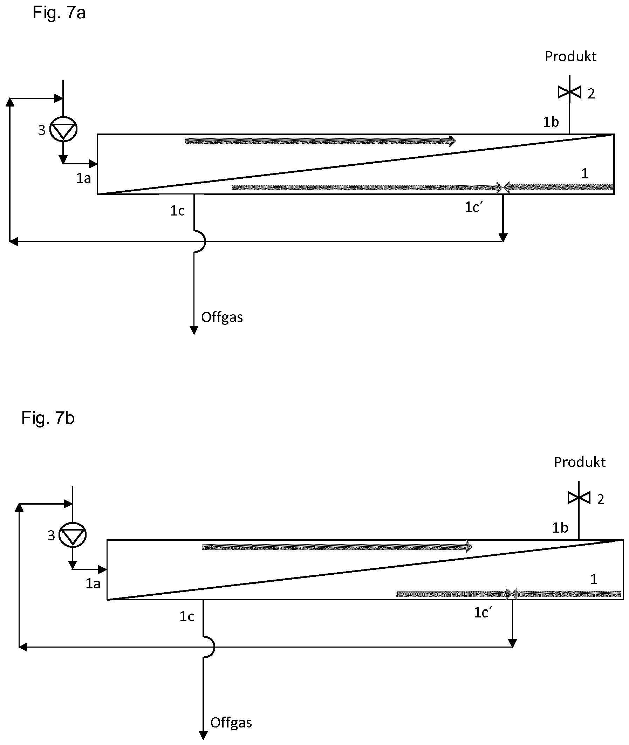

Aus Figur 7a, in Anlehnung an Figur 1, ist leicht ersichtlich, dass bei 1c' austretendes bzw. abgesaugtes Permeat mehrheitlich (d.h. mehr als die Hälfte) von Bereichen der Membran stammt, welche im Gleichstrom betrieben wurden.

Wenn nun ausgehend von der in Figur 7a dargestellten Situation die Drehzahl des Verdichters (3) verringert wird, verändert sich auch die Zusammensetzung des bei 1c' austretendes bzw. abgesaugtes Permeats, das Permeat wird dann weniger von Bereichen der Membran stammen, welche im Gleichstrom betrieben wurden (siehe Figur 7b).

Wie bereits ausgeführt dient die Druckregeleinrichtung dabei dazu, in der erfindungsgemäßen Membraneinheit retentatseitig einen Widerstand vorzusehen, ohne Druckregeleinrichtung (2) wäre im Retentatraum kein Gegendruck vorhanden und eine Trennung nicht möglich. Druckerhöhungen im Retentatraum werden erfindungsgemäß zumeist über Drehzahlerhöhungen des Verdichters (3) vorgesehen, wodurch sich auch die Menge an in die Membraneinheit zugeführtem Feedgas verändert.

Darüber hinaus ist dem Fachmann auch klar, dass an einer erfindungsgemäß verwendeten Trennmembran immer ein Konzentrationsgefälle bezüglich des durch die Membran hindurchtretenden Methans gegeben ist, im rechten Teil der Membran (nahe dem Retentatausgang 1b) ist das Permeat im Permeatraum jedenfalls methanreicher als im linken Teil der Membran (nahe dem Feedgaseingang 1a).

Wenn in der erfindungsgemäßen Membraneinheit retentatseitig ein Widerstand erzeugt wird (beispielsweise indem das Regelventil (2) weiter geschlossen wird, wodurch sich im Retentatraum Druck aufbaut) und der Permeatausgang (1c') der Membraneinheit (1) ansaugseitig mit dem Verdichter (3) bzw. der in den Verdichter führenden Gaszufuhr leitungsmäßig verbunden ist, wird Permeat über den Permeatausgang (1c') abgezogen und dem Feedgas wieder zugemengt. Die Zusammensetzung des Permeats welches über den Permeatausgang (1c') abgezogen wird kann dabei durch die gewählte Drehzahl des Verdichters (3) beeinflusst werden. Durch Erhöhung der Drehzahl vergrößert sich die Menge an Permeat welches von Bereichen der Membran stammt, die im Gleichstrom betrieben werden (Figur 7a), bei Verringerung der Drehzahl verringert sich entsprechend die Menge an Permeat welches von Bereichen der Membran stammt die im Gleichstrom betrieben werden (Figur 7b).

Da die Gesamtmenge an durch die Membran hindurchtretendem Permeat praktisch konstant gehalten werden kann (eine durch Erhöhung der Drehzahl des Verdichters (3) verursachte Druckerhöhung im Retentatraum und eine damit verbundene geänderte Trennleistung der Membran kann durch entsprechendes Öffnen der Druckregeleinrichtung (2) und damit Verminderung des Widerstands ausgeglichen werden), kann die Zusammensetzung des Offgases hinsichtlich seines Methangehalts erfindungsgemäß über die Drehzahl des Verdichters (3) gesteuert werden.

Der erfinderische Effekt, dessen Auftreten allerdings auf Methankonzentrationen im Feedgas von nicht über 30 Vol.-% beschränkt ist, wird also nicht allein über die Druckregeleinrichtung (2) erzielt, diese dient nur dazu, im Retentatraum der erfindungsgemäßen Membraneinheit einen Widerstand vorzusehen. Der gewünschte Effekt wird über die Drehzahl des Verdichters (3), gegebenenfalls in Kombination mit der Druckregeleinrichtung (2), erzielt.

## Patentansprüche

1. Vorrichtung zur Auftrennung eines Gasgemisches in Produktgas und Offgas mittels Gaspermeation mit einer Membraneinheit (1) und einem der Membraneinheit (1) vorgeschalteten, vorzugsweise drehzahlregelbaren Verdichter (3), welche Membraneinheit (1) einen Gaseingang (1a), einen Retentat- bzw. Produktgasausgang (1b) und einen Permeat- bzw. Offgasausgang (1c) aufweist, wobei die Membraneinheit (1) mindestens einen weiteren, vom Gaseingang (1a) stromabwärts liegenden Permeatausgang (1c') aufweist und der Permeatausgang (1c') der Membraneinheit (1) ansaugseitig mit dem Verdichter (3) bzw. der in den Verdichter führenden Gaszufuhr leitungsmäßig verbunden ist, **dadurch gekennzeichnet, dass** am oder nach dem Retentatausgang (1b) eine Druckregeleinrichtung (2) vorgesehen ist und ein Gasgemisch mit einem Anteil von mehr als 50 Vol.-%, vorzugsweise mehr als 55 Vol.-%, 60 Vol.-%, 65 Vol.-%, 70 Vol.-%, 75 Vol.-%, 80 Vol.-%, 85 Vol.-%, insbesondere bevorzugt mehr als 90 Vol.-% bzw. 95 Vol.-% CH₄/CO₂ im Gasgemisch und mit einer Methankonzentration nicht über 30 Vol.-% eingesetzt wird, wobei der Abzug des Permeats über den Permeatausgang (1c') in Abhängigkeit von der Konzentration von Produktgas im Offgas gesteuert bzw. geregelt wird.

2. Vorrichtung nach Anspruch 1, **dadurch gekennzeichnet, dass** am oder nach dem ersten Permeatausgang (1c) ein vorzugsweise drehzahlregelbarer Verdichter (4) und/oder eine Druckregeleinrichtung (5) vorgesehen ist bzw. sind.

3. Vorrichtung nach Anspruch 1 oder 2, **dadurch gekennzeichnet, dass** in der Leitung vom Permeatausgang (1c') der Membraneinheit (1) ansaugseitig zum Verdichter (3) bzw. der in den Verdichter führenden Gaszufuhr eine Druckregeleinrichtung (6) vorgesehen ist.

4. Vorrichtung nach Anspruch 1, **dadurch gekennzeichnet, dass** der drehzahlregelbare Verdichter (3) und die Druckregeleinrichtung (2) mit einer Steuereinrichtung (7) verbunden sind.

5. Vorrichtung nach Anspruch 2 oder 4, **dadurch gekennzeichnet, dass** der vorzugsweise drehzahlregelbare Verdichter (4) und/oder die Druckregeleinrichtung (5) mit einer bzw. der Steuereinrichtung (7) verbunden sind.

6. Vorrichtung nach Anspruch 3 oder 5, **dadurch gekennzeichnet, dass** die Druckregeleinrichtung (6) mit einer bzw. der Steuereinrichtung (7) verbunden ist.

7. Verwendung einer Vorrichtung nach einem der Ansprüche 1 bis 6 zur Trennung eines Gasgemisches mit einem Anteil im Gasgemisch von mehr als 50 Vol.-%, vorzugsweise mehr als 55 Vol.-%, 60 Vol.-%, 65 Vol.-%, 70 Vol.-%, 75 Vol.-%, 80 Vol.-%, 85 Vol.-%, insbesondere bevorzugt mehr als 90 Vol.-% bzw. 95 Vol.-% CH₄/CO₂ und mit einer Methankonzentration nicht über 30 Vol.-% in CH₄ als Produktgas und CO₂ als Offgas.

8. Verfahren zur Auftrennung eines Gasgemisches mit einem Anteil im Gasgemisch von mehr als 50 Vol.-%, vorzugsweise mehr als 55 Vol.-%, 60 Vol.-%, 65 Vol.-%, 70 Vol.-%, 75 Vol.-%, 80 Vol.-%, 85 Vol.-%, insbesondere bevorzugt mehr als 90 Vol.-% bzw. 95 Vol.-% CH_{4/}CO₂ und mit einer Methankonzentration nicht über 30 Vol.-% in Produktgas und Offgas mittels Gaspermeation, **dadurch gekennzeichnet, dass** das Permeat einer Membraneinheit (1) teilweise im Gleichstrom und teilweise im Gegenstrom gewonnen wird, wobei das im Gleichstrom gewonnene Permeat druckbeaufschlagt und der Membraneinheit (1) wieder als Feedgas zugeführt wird, das im Gegenstrom gewonnene Permeat als Offgas abgezogen wird, und weiters das Retentat der Membraneinheit (1) über den Retentatausgang (1b) der Membraneinheit (1) und eine dort vorgesehene Druckregeleinrichtung (2) als Produktgas abgezogen wird, wobei der Abzug des im Gleichstrom gewonnenen Permeats in Abhängigkeit von der Konzentration von Produktgas im Offgas gesteuert bzw. geregelt wird.

9. Verfahren nach Anspruch 8, **dadurch gekennzeichnet, dass** der Druck des Retentats in der Membraneinheit (1) erhöht wird.

## Claims

1. Apparatus for separating a gas mixture into a product gas and an off-gas by means of gas permeation using a membrane unit (1) and a compressor (3) which is arranged upstream of the membrane unit (1) and of which the rotational speed can preferably be controlled in a closed-loop manner, which membrane unit (1) comprises a gas inlet (1a), a retentate or product gas outlet (1b) and a permeate or off-gas outlet (1c), the membrane unit (1) comprising at least one additional permeate outlet (1c') that is downstream of the gas inlet (1a) and the permeate outlet (1c') of the membrane unit (1) being connected on the intake side to the compressor (3) or to the gas supply leading into the compressor by means of lines, **characterised in that** a closed-loop pressure control device (2) is provided at or downstream of the retentate outlet (1b) and a gas mixture having a proportion of CH₄/CO₂ in the gas mixture of more than 50 vol.%, preferably more than 55 vol.%, 60 vol.%, 65 vol.%, 70 vol.%, 75 vol.%, 80 vol.%, 85 vol.%, particularly preferably more than 90 vol.% or 95 vol.%, and having a methane concentration of no more than 30 vol.% is used, the discharge of the permeate via the permeate outlet (1c') being controlled in an open-loop or closed-loop manner depending on the concentration of product gas in the off-gas.

2. Apparatus according to claim 1, **characterised in that** a compressor (4), of which the rotational speed can preferably be controlled in a closed-loop manner, and/or a closed-loop pressure control device (5) is or are provided at or downstream of the first permeate outlet (1c).

3. Apparatus according to either claim 1 or claim 2, **characterised in that** a closed-loop pressure control device (6) is provided in the line from the permeate outlet (1c') of the membrane unit (1) on the intake side to the compressor (3) or in the gas supply line leading into the compressor.

4. Apparatus according to claim 1, **characterised in that** the compressor (3), of which the rotational speed can be controlled in a closed-loop manner, and the closed-loop pressure control device (2) are connected to an open-loop control device (7).

5. Apparatus according to either claim 2 or claim 4, **characterised in that** the compressor (4), of which the rotational speed can preferably be controlled in a closed-loop manner, and/or the closed-loop pressure control device (5) are connected to a or the open-loop control device (7).

6. Apparatus according to either claim 3 or claim 5, **characterised in that** the closed-loop pressure control device (6) is connected to a or the open-loop control device (7).

7. Use of an apparatus according to any of claims 1 to 6 for separating a gas mixture having a proportion of CH₄/CO₂ in the gas mixture of more than 50 vol.%, preferably more than 55 vol.%, 60 vol.%, 65 vol.%, 70 vol.%, 75 vol.%, 80 vol.%, 85 vol.%, particularly preferably more than 90 vol.% or 95 vol.%, and having a methane concentration of no more than 30 vol.% into CH₄ as the product gas and CO₂ as the off-gas.

8. Method for separating a gas mixture having a proportion of CH₄/CO₂ in the gas mixture of more than 50 vol.%, preferably more than 55 vol.%, 60 vol.%, 65 vol.%, 70 vol.%, 75 vol.%, 80 vol.%, 85 vol.%, particularly preferably more than 90 vol.% or 95 vol.%, and having a methane concentration of no more than 30 vol.% into product gas and off-gas by means of gas permeation, **characterised in that** the permeate of a membrane unit (1) is obtained in part in parallel flow and in part in counterflow, the permeate obtained in parallel flow being pressurised and supplied to the membrane unit (1) again as feed gas, the permeate obtained in counterflow being discharged as the off-gas, and furthermore the retentate of the membrane unit (1) being discharged via the retentate outlet (1b) of the membrane unit (1) and via a closed-loop pressure control device (2) provided at said outlet as the product gas, the discharge of the permeate obtained in parallel flow being controlled in an open-loop or closed-loop manner depending on the concentration of product gas in the off-gas.

9. Method according to claim 8, **characterised in that** the pressure of the retentate in the membrane unit (1) is increased.

## Revendications

1. Dispositif pour la séparation d'un mélange gazeux en gaz produit et gaz rejeté par perméation de gaz avec une unité à membrane (1) et un compresseur (3) branché en amont de l'unité à membrane (1), de préférence réglable en ce qui concerne la vitesse de rotation, laquelle unité à membrane (1) présente une entrée de gaz (1a), une sortie de rétentat ou de gaz produit (1b) et une sortie de perméat ou de gaz rejeté (1c), où l'unité à membrane (1) présente au moins une autre sortie de perméat (1c') située en aval de l'entrée de gaz (1a) et la sortie de perméat (1c') de l'unité à membrane (1) est reliée par une conduite du côté de l'aspiration avec le compresseur (3) ou l'alimentation en gaz qui conduit au compresseur, **caractérisé en ce qu'**il est prévu une installation de régulation de la pression (2) sur ou après la sortie de rétentat (1b) et un mélange gazeux avec une proportion de plus de 50 % en volume, de préférence plus de 55 % en volume, 60 % en volume, 65 % en volume, 70 % en volume, 75 % en volume, 80 % en volume, 85 % en volume, de manière particulièrement préférée plus de 90 % en volume ou 95 % en volume de CH_{4/}CO₂ dans le mélange gazeux et avec une concentration en méthane ne dépassant pas 30 % en volume est utilisé, où le soutirage du perméat par le biais de la sortie de perméat (1c') est commandé ou régulé en fonction de la concentration de gaz produit dans le gaz rejeté.

2. Dispositif selon la revendication 1, **caractérisé en ce qu'**il est prévu sur ou après la première sortie de perméat (1c) un compresseur (4) de préférence réglable en ce qui concerne la vitesse de rotation et/ou une installation de régulation de la pression (5).

3. Dispositif selon la revendication 1 ou 2, **caractérisé en ce qu'**il est prévu une installation de régulation de la pression (6) dans la conduite allant de la sortie de perméat (1c') de l'unité à membrane (1) au compresseur (3) du côté de l'aspiration ou l'alimentation en gaz conduisant au compresseur.

4. Dispositif selon la revendication 1, **caractérisé en ce que** le compresseur (3) réglable en ce qui concerne la vitesse de rotation et l'installation de régulation de la pression (2) sont reliés à une installation de commande (7).

5. Dispositif selon la revendication 2 ou 4, **caractérisé en ce que** le compresseur (4) de préférence réglable en ce qui concerne la vitesse de rotation et/ou l'installation de régulation de la pression (5) sont reliés à une ou l'installation de commande (7).

6. Dispositif selon la revendication 3 ou 5, **caractérisé en ce que** l'installation de régulation de la pression (6) est reliée à une ou l'installation de commande (7).

7. Utilisation d'un dispositif selon l'une des revendications 1 à 6 pour la séparation d'un mélange gazeux avec une proportion dans le mélange gazeux de plus de 50 % en volume, de préférence plus de 55 % en volume, 60 % en volume, 65 % en volume, 70 % en volume, 75 % en volume, 80 % en volume, 85 % en volume, de manière particulièrement préférée plus de 90 % en volume ou 95 % en volume de CH_{4/}CO₂ et avec une concentration en méthane ne dépassant pas 30 % en volume en CH₄ comme gaz produit et CO₂ comme gaz rejeté.

8. Procédé pour la séparation d'un mélange gazeux avec une proportion dans le mélange gazeux de plus de 50 % en volume, de préférence plus de 55 % en volume, 60 % en volume, 65 % en volume, 70 % en volume, 75 % en volume, 80 % en volume, 85 % en volume, de manière particulièrement préférée plus de 90 % en volume ou 95 % en volume de CH_{4/}CO₂ et avec une concentration en méthane ne dépassant pas 30 % en volume en gaz produit et gaz rejeté par perméation de gaz, **caractérisé en ce que** le perméat d'une unité à membrane (1) est obtenu en partie à co-courant et en partie à contrecourant, où le perméat obtenu à co-courant est soumis à une pression et envoyé de nouveau à l'unité à membrane (1) comme gaz d'alimentation, le perméat obtenu à contrecourant est soutiré comme gaz rejeté, et en outre le rétentat de l'unité à membrane (1) est soutiré comme gaz produit par le biais de la sortie de rétentat (1b) de l'unité à membrane (1) et d'une installation de régulation de la pression (2) qui est prévue dans celle-ci, où le soutirage du perméat obtenu à co-courant est commandé ou régulé en fonction de la concentration de gaz produit dans le gaz rejeté.

9. Procédé selon la revendication 8, **caractérisé en ce que** la pression du rétentat dans l'unité à membrane (1) est augmentée.
